# EUROPEAN PATENT APPLICATION

(11) **EP 4 202 012 A2**
(43) Date of publication of application: **28.06.2023**
(21) Application number: 22215847.9
(22) Date of filing: 22.12.2022
(51) Int. Cl.: C09K 19/18, C09K 19/12, C09K 19/30

(54) **LIQUID CRYSTAL COMPOUNDS AND COMPOSITIONS FOR TUNABLE LENSES**

(30) Priority: 23.12.2021 US 202163293528 P
(71) Applicant: Apple Inc., Cupertino CA 95014 (US)
(72) Inventor: DENG, Huiyang, Cupertino, CA95014 (US); Jiang, Jiaqi, Cupertino, 95014 (US); Koller, Jeffrey G., Cupertino, 95014 (US)
(74) Representative: Barnfather, Karl Jon

(57) **Abstract**

The disclosure is generally directed to compounds and compositions that can be used as liquid crystal materials in adjustable ophthalmic lenses.

## Description

### Priority

This patent application claims the benefit of U.S. Provisional Patent Application No. 63/293,528, entitled "LIQUID CRYSTAL COMPOUNDS AND COMPOSITIONS FOR TUNABLE LENSES," filed on December 23, 2021, which is incorporated herein by reference in its entirety.

### Technical Field

The disclosure generally relates to liquid crystal materials having compounds and compositions that can be used in tunable lenses.

### Background

Ophthalmic lenses are ophthalmic devices that alter or change the visual powers of the human eye. Adjustable ophthalmic lenses can be tuned to change visible properties of light passing therethrough. Tunable lenses can include one or more liquid crystal cells. Each liquid crystal cell can include a layer of liquid crystal material interposed between transparent substrates. Control circuitry can apply control signals to an array of electrodes in the liquid crystal cell to adjust a phase profile of the liquid crystal material. In various arrangements, an adjustable lens can include multiple liquid crystal cells (e.g., three or six liquid crystal cells). The electrodes in the liquid crystal cells can be oriented along three different directions.

### Summary

Additional embodiments and features are set forth in part in the description that follows, and in part will become apparent to those skilled in the art upon examination of the specification, or can be learned by the practice of the embodiments discussed herein. A further understanding of the nature and advantages of certain embodiments can be realized by reference to the remaining portions of the specification and the drawings, which forms a part of this disclosure.

In a first aspect, the disclosure is directed to a compound having the structure of Formula (I): 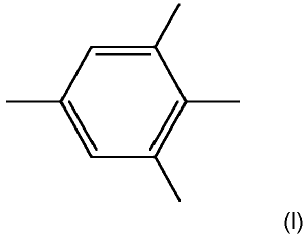 wherein
R₁ is selected from hydrogen, a saturated C₁₋₁₀ alkyl, halogen, and pseudohalogen;
R₂ and R₃ are each independently selected from hydrogen, a halogen, and a pseudohalogen;
R₄ is selected from Formula (II) and Formula (III): 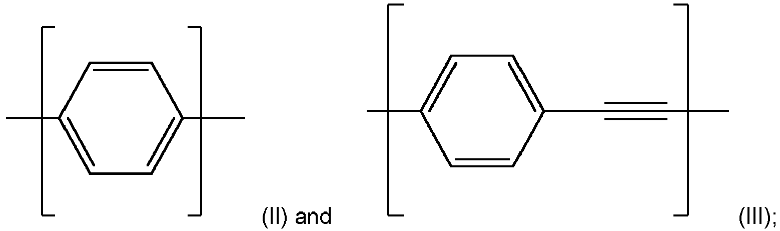
wherein m is an integer from 1 to 5;
R₅ is saturated C₁-C₁₀ alkyl or saturated C₁-C₁₀ alkoxy;
n is an integer from 1 to 5; and
R₆ is a saturated C₁-C₁₀ alkyl or saturated C₁-C₁₀ alkoxy.

In a second aspect, the disclosure is directed to a composition comprising multiple compounds.

In a third aspect, the disclosure is directed to a first compound having the structure of Formula (IV): 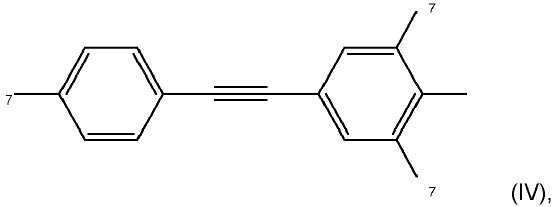
a second compound having the structure of Formula (V): 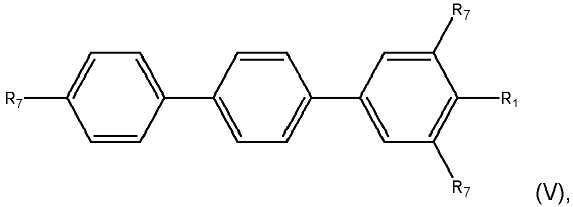 and
a third compound having the structure of Formula (VI): 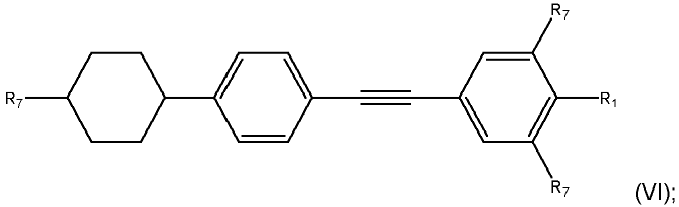 wherein
   R₁ is selected from saturated C₁₋₁₀ alkyl, halogen, and pseudohalogen;
   R₂ and R₃ are each independently selected from hydrogen, a halogen, and a pseudohalogen;
   R₇ is saturated C₁-C₁₀ alkyl or saturated C₁-C₁₀ alkoxy;
   R₈ is saturated C₁-C₁₀ alkyl or saturated C₁-C₁₀ alkoxy; and
   R₉ is saturated C₁-C₁₀ alkyl.

In a fourth aspect, the disclosure is directed to a liquid crystal cell a first substrate transparent in the visible spectrum and a second substrate transparent in the visible spectrum on opposing sides of a liquid crystal layer. The liquid crystal layer includes a compound or a composition as described herein.

### Detailed Description

The disclosure can be understood by reference to the following detailed description, taken in conjunction with the drawings as described below. It is noted that, for purposes of illustrative clarity, certain elements in various drawings may not be drawn to scale, can be represented schematically or conceptually, or otherwise may not correspond exactly to certain physical configurations of embodiments.

The disclosure is directed to compounds and compositions that can be used as liquid crystal materials in ophthalmic lenses. In some variations, the compounds can be used as liquid crystal materials, or can be combined into compositions that are used as liquid crystal materials. The compounds have a rigid core comprising one or more phenyl groups linked by a single bond or a pi-electron containing bridge group, a polar group linked to a terminal phenyl substituent of the rigid core, a non-polar terminal group opposite the rigid phenyl core, and optionally one or more transverse substituents. Compositions are combinations of different individual compounds.

### Definitions

"Alkyl" by itself or as part of another substituent refers to a saturated or unsaturated branched, straight-chain, or cycloalkyl radical derived by the removal of one hydrogen atom from a single carbon atom of a parent alkane. Typical alkyl groups include, but are not limited to, methyl, ethyl, propyl (such as propan-1-yl, propan-2-yl (isopropyl), cyclopropan-1-yl, etc.), butyl (such as butan-1-yl, butan-2-yl (sec-butyl), 2-methyl-propan-I-yl (isobutyl), 2-methyl-propan-2-yl (tert-butyl), cyclobutan-1-yl, etc.) and the like. Typical cycloalkyl groups include cyclopentyl, cyclohexyl, cycloheptyl, cyclononyl, cyclodecyl, and the like.

"Alkoxy" refers to a radical -OR where R represents an alkyl group as defined herein. Representative examples include, but are not limited to, methoxy, ethoxy, propoxy, butoxy, cyclohexyloxy and the like.

"Compounds" disclosed herein include any specific compounds within a formula. Compounds can be identified either by their chemical structure and/or chemical name. The compounds described herein can comprise one or more chiral centers and/or double bonds and therefore can exist as stereoisomers such as double bond isomers (i.e., geometric isomers), enantiomers, or diastereomers. Accordingly, any chemical structures within the scope of the specification depicted, in whole or in part, with a relative configuration encompass all possible enantiomers and stereoisomers of the illustrated compounds including the stereoisomerically pure form (e.g., geometrically pure, enantiomerically pure, or diastereomerically pure) and enantiomeric and stereoisomeric mixtures. Enantiomeric and stereoisomeric mixtures can be resolved into their component enantiomers or stereoisomers using separation techniques or chiral synthesis techniques well known to those skilled in the art. Compounds include, for example, optical isomers of compounds, racemates thereof, and other mixtures thereof. In such embodiments, a single enantiomer or diastereomer, i.e., optically active form can be obtained by asymmetric synthesis or by resolution of the racemates. Resolution of the racemates can be accomplished, for example, by methods such as crystallization in the presence of a resolving agent, or chromatography using, for example, chiral stationary phases.

Compounds also include isotopically labeled compounds where one or more atoms have an atomic mass different from the atomic mass conventionally found in nature. Examples of isotopes that can be incorporated into the compounds disclosed herein include, for example, ²H, ³H, ¹¹C, ¹³C, ¹⁴C, ¹⁵N, ¹⁸O, ¹⁷O, ³³S, ³⁴S, etc. Compounds can exist in unsolvated forms as well as solvated forms, including hydrated forms and as N oxides. In general, compounds disclosed herein can be free acid, hydrated, solvated, or N oxides. Compounds include salts thereof, solvates of the free acid form of any of the foregoing. A solvate refers to a molecular complex of a compound with one or more solvent molecules in a stoichiometric or non-stoichiometric amount. When partial structures of the compounds are illustrated, an asterisk (*) indicates the point of attachment of the partial structure to the rest of the molecule.

"Halogen" refers to a fluoro, chloro, bromo, or iodo group.

"Pseudohalogen" refers to a polyatomic anion that can be substituted for a halogen. Non-limiting examples of pseudohalogens include, but are not limited to, cyanide, cyanate, thiocyanate, and azide.

### Brief Description of the Drawings

Non-limiting aspects of the disclosure are described by reference to the drawings and descriptions. FIGS. 1-6 are merely illustrative, and provide non-limiting examples of variations of the disclosure.
FIG. 1 is a side view of an illustrative liquid crystal cell that can be used to form an adjustable lens, in accordance with an illustrative embodiment;
FIG. 2 is a side view of an illustrative liquid crystal module having first and second liquid crystal layers with antiparallel liquid crystal alignment orientations, in accordance with an illustrative embodiment;
FIG. 3 is a side view of light incident on a liquid crystal module, in accordance with an illustrative embodiment;
FIG. 4 is an exploded perspective view of an illustrative adjustable lens having first, second, and third liquid crystal cells, each with an associated orientation of electrodes, in accordance with an illustrative embodiment;
FIG. 5 is an exploded perspective view of an illustrative adjustable lens having first, second, and third liquid crystal modules, each with an associated orientation of electrodes, in accordance with an illustrative embodiment; and
Figure 6 depicts the band gap of the change in polarizability for several example compounds, in accordance with illustrative embodiments.

### Ophthalmic Lenses

The disclosure is directed to ophthalmic lenses having liquid crystal compounds or compositions included herein.

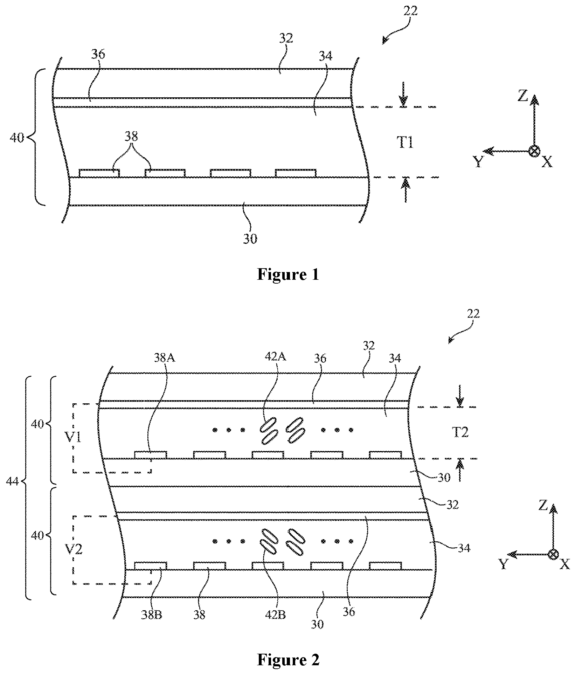

A cross-sectional side view of an illustrative ophthalmic lens is shown in FIG. 1. Component 22 can include liquid crystal cell 40. Liquid crystal cell 40 can have a liquid crystal layer 34 comprising compounds or compositions of the disclosure. Liquid crystal layer 34 can be interposed between substrates transparent in the visible spectrum (400 nm - 750 nm) such as upper substrate 32 and lower substrate 30. Substrates 32 and 30 can be formed from transparent material such as clear glass, sapphire, or other transparent crystalline material, cellulose triacetate, transparent plastic, cyclic olefin polymers (COPs), cyclic olefin copolymers (COCs), or other transparent layers. Component 22 can have a pattern of electrodes that can be supplied with signals from control circuitry 26 to produce desired voltages on component 22. In the example of FIG. 1, these electrodes include elongated electrodes (e.g., strip-shaped electrodes) such as electrodes 38 on substrate 30 that run along the X dimension and a common electrode such as common electrode 36 on substrate 32 (e.g., a blanket layer of conductive material on substrate 32). Electrodes 36 and 38 can be formed from transparent conductive material such as indium tin oxide, conductive polymers such as poly(3,4-ethylenedioxythiophene) polystyrene sulfonate (PEDOT:PPS), or other transparent electrode structures and can be located on outer and/or inner surfaces of substrates 32 and 30.

At each location of electrode strips 38 in component 22, a voltage can be applied across liquid crystal layer 34 by supplying a first voltage to electrode 38 and a second voltage (e.g., a ground voltage) to common electrode 36. The liquid crystal between the two electrodes will receive an applied electric field with a magnitude that is proportional to the difference between the first and second voltages on the electrodes. By controlling the voltages on electrodes 38 and common electrode 36, the index of refraction of liquid crystal layer 34 of component 22 can be dynamically adjusted to produce customized lenses.

In the example of FIG. 1, strip-shaped electrodes 38 (sometimes referred to as finger electrodes) extend parallel to the X-axis. This allows the index-of-refraction profile (sometimes referred to as the phase profile) of liquid crystal cell 40 to be modulated in the Y-dimension by applying the desired voltages to each finger electrode 38.

When an electric field is applied to the liquid crystals of layer 34, the liquid crystals change orientation. The speed at which a given liquid crystal material can be reoriented is limited by factors such as the thickness of layer 34 (e.g., thickness T1 of FIG. 1, sometimes referred to as the cell gap), or viscosity and/or elasticity of the liquid crystal compound or composition.

Lens component 22 can include two or more liquid crystal cells 40 stacked on top of one another. This type of arrangement is illustrated in FIG. 2.

As shown in FIG. 2, ophthalmic lens component 22 can include liquid crystal module 44. Liquid crystal module 44 can include two or more liquid crystal cells 40. Each liquid crystal cell can include liquid crystal layer 34 that includes a compound or composition of the disclosure interposed between upper substrate 32 and lower substrate 30. In some variations, at least one of the liquid crystal layers includes a compound or composition described herein. Different liquid crystal layers can have the same or different compound or composition. Finger electrodes 38 can be formed on each lower substrate 30 and can extend parallel to the X-axis. Common electrode 36 can be formed on each upper substrate 32. If desired, common voltage electrode 36 can be formed on lower substrate 30 and finger electrodes 38 can be formed on upper substrate 32.

The cell gap of each liquid crystal cell 40 in module 44 can be less than that of liquid crystal cell 40 of FIG. 1. For example, liquid crystal layers 34 of module 44 in FIG. 2 can each have a thickness T2, which is less than thickness T1 of liquid crystal layer 34 in cell 40 of FIG. 1. The reduced cell gap can improve the tuning speed of liquid crystal layers 34 while still maintaining a satisfactory tuning range.

At each location of finger electrode 38 in component 22, a voltage can be applied across each liquid crystal layer 34 by supplying a first voltage to finger electrode 38 and a second voltage (e.g., a ground voltage) to common electrode 36. The liquid crystal between the two electrodes will receive an applied electric field with a magnitude that is proportional to the difference between the first and second voltages on the electrodes. By controlling the voltages on electrodes 38 and common electrode 36, the index of refraction of each liquid crystal layer 34 of component 22 can be dynamically adjusted to produce customized lenses. Because finger electrodes 38 extend along the X-dimension, the phase profile of each liquid crystal cell 40 can be modulated in the Y-dimension by applying the desired voltages to each finger electrode 38.

Overlapping portions of the two liquid crystal layers 34 in module 44 can be controlled using the same or different voltages to achieve the desired index of refraction at that portion of module 44. For example, finger electrode 38A of upper liquid crystal cell 40 in module 44 can overlap finger electrode 38B of lower liquid crystal cell 40 in module 44. A first voltage V1 can be applied across a portion of upper liquid crystal layer 34 overlapping finger electrode 38A, and a second voltage V2 can be applied across a portion of lower liquid crystal layer 34 overlapping finger electrode 38B. Voltages V1 and V2 can be different or can be the same. Control circuitry 26 can determine the ratio of V1 to V2 based on the desired index of refraction at that portion of the liquid crystal module 44.

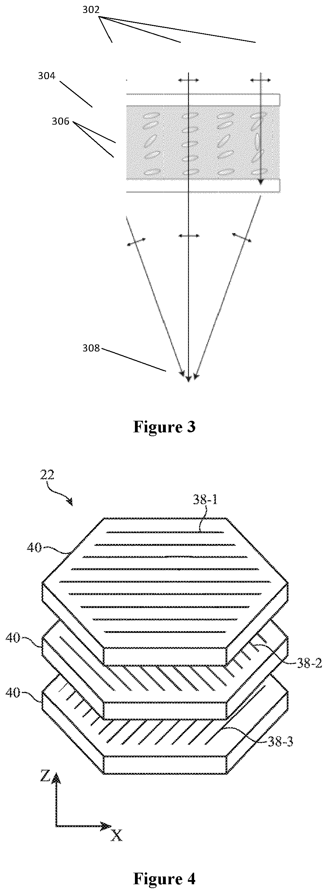

FIG. 3 depicts a cross-sectional side view of an illustrative of light 302 incident on a liquid crystal module 304. The liquid crystal molecules 306 in the liquid crystal module 304 can be tuned such that the light can focus with a tunable focal length 308 after passing through the lens.

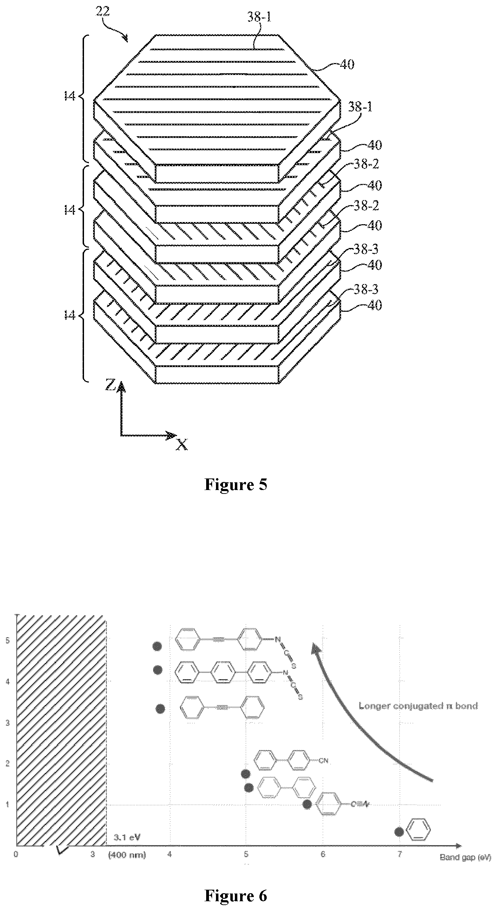

FIGS. 4 and 5 show exploded perspective views of illustrative lens components 22 with three orientations of electrodes. In the example of FIG. 4, adjustable lens components 22 include three liquid crystal cells 40. Each liquid crystal cell 40 can have a structure of the type described in connection with FIG. 1, with finger electrodes 38-1, 38-2, and 38-3 oriented along three different directions. For example, finger electrodes 38-1 can be oriented at 0 degrees relative to the X-axis, finger electrodes 38-2 can be oriented at 120 degrees relative to the X-axis, and finger electrodes 38-3 can be oriented at 60 degrees relative to the X-axis. This is merely illustrative, however. In general, electrodes 38-1, 38-2, and 38-3 can have any suitable orientation.

In FIG. 5, adjustable lens components 22 include three liquid crystal modules 14. Each liquid crystal module 14 can have a structure of the type described in connection with FIG. 2. In particular, each liquid crystal module 14 can include an upper liquid crystal cell 40 and a lower liquid crystal cell 40. The liquid crystal layers of the upper and lower liquid crystal cells 40 may, if desired, have antiparallel liquid crystal alignment orientations. As shown in FIG. 5, finger electrodes 38-1, 38-2, and 38-3 of liquid crystal modules 14 are oriented along three different directions. For example, finger electrodes 38-1 can be oriented at 0 degrees relative to the X-axis, finger electrodes 38-2 can be oriented at 120 degrees relative to the X-axis, and finger electrodes 38-3 can be oriented at 60 degrees relative to the X-axis. This is merely illustrative, however. In general, electrodes 38-1, 38-2, and 38-3 can have any suitable orientation.

In various aspects, the compounds and compositions of the disclosure can be used in the liquid crystal lenses described in U.S. Patent No. 11,086,143, which is incorporated herein by reference in its entirety.

The disclosure is directed to devices including a tunable liquid crystal lens. In various aspects, liquid crystals can be designed to have properties including but not limited to a lower viscosity, higher elastic constant (K33), and more polarizable compounds. Liquid crystal lenses using the compounds and compositions as liquid crystal materials thereby provide for improve tunable lenses in the visible spectrum (400 nm - 750 nm). The lenses can be used in, for example, automated presbyopia glasses and AR/VR glasses.

### Compounds

Liquid crystal cells described herein include a liquid crystal layer comprising the compounds or compositions of the disclosure.

In one variation, the compound has the structure of formula (I): 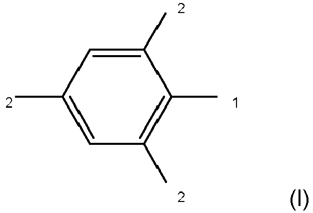 wherein
R₁ is selected from a hydrogen, saturated C₁₋₁₀ alkyl, halogen, and pseudohalogen;
R₂ and R₃ are each independently selected from hydrogen, a halogen, and a pseudohalogen;
R₄ is selected from the structure of Formula (II) or Formula (III): 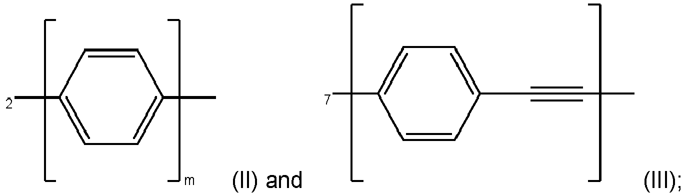
m is an integer from 1 to 5;
R₅ is saturated C₁-C₁₀ alkyl or saturated C₁-C₁₀ alkoxy;
n is an integer from 1 to 5; and
R₆ is saturated C₁-C₁₀ alkyl or saturated C₁-C₁₀ alkoxy.

The substituents can be selected from a range of possible options.

In some variations, R₁ is hydrogen. In some variations, R₁ is a saturated C₁₋₁₀ alkyl. In some variations, R₁ is a saturated C₁₋₅ alkyl. In some variations, R₁ is a saturated C₁₋₂ alkyl. In some variations, R₁ is ethyl. In some variations, R₁ is methyl. In some variations, R₁ is a halogen. In some variations, R₁ is a pseudohalogen. In some further variations, R₁ is a cyano substituent. In some further variations, R₁ is a thioisocyanate substituent.

In some variations, R₂ and R₃ are each independently selected from hydrogen or a halogen. In some variations, R₂ and R₃ are both hydrogen. In some variations, R₂ and R₃ are both a halogen. In some variations, the halogen is fluorine. In some variations, R₂ and R₃ are both fluorine. In some variations, R₂ is hydrogen and R₃ is a halogen.

When R₄ is Formula (II), in some variations m is 1. In some variations, m is 2. In some variations, m is 3. In some variations, m is 4. In some variations, m is 5. Further, when R₄ is Formula (II), R₅ is a saturated alkyl. In some variations, R₅ is a saturated straight chain C₁-C₁₀ alkyl. In some variations, R₅ is a saturated straight chain C₁-C₅ alkyl. In some variations, R₅ is n-butyl. In some variations, R₅ is n-propyl. In some variations, R₅ is ethyl. In some variations, R₅ is methyl. In some variations, R₅ is a C₅-C₁₀ cycloalkyl. In some variations, R₅ is a saturated C₅-C₁₀ cycloalkyl. In some variations, R₅ is cyclohexyl. In some variations, R₅ is a saturated C₁-C₁₀ alkoxy. In some variations, R₅ is a saturated C₁-C₅ alkoxy.

When R₄ is Formula (III), in some variations n is 1. In some variations, n is 2. In some variations, n is 3. In some variations, n is 4. In some variations, n is 5. Further, when R₄ is Formula (III), R₆ is a saturated alkyl. In some variations, R₆ is a saturated straight chain C₁-C₁₀ alkyl. In some variations, R₆ is a saturated straight chain C₁-C₅ alkyl. In some variations, R₆ is n-butyl. In some variations, R₆ is n-propyl. In some variations, R₆ is ethyl. In some variations, R₅ is methyl. In some variations, R₆ is a C₅-C₁₀ cycloalkyl. In some variations, R₆ is a saturated C₅-C₁₀ cycloalkyl. In some variations, R₆ is cyclohexyl. In some variations, R₆ is a saturated C₁-C₁₀ alkoxy. In some variations, R₆ is a saturated C₁-C₅ alkoxy. In various aspects, R₄ can be combined with any structure or variable herein, in any combination.

In some variations, the compound has the structure of Formula (IV): 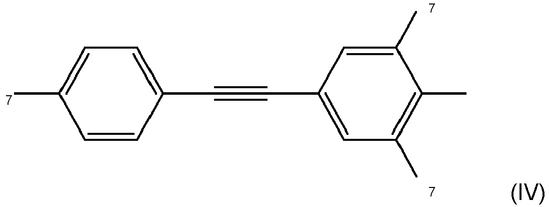 wherein R₁, R₂, and R₃ can be as described herein, in any combination, and R₇ is a saturated alkyl. In some variations, R₇ is a saturated straight chain C₁-C₁₀ alkyl. In some variations, R₇ is a saturated straight chain C₁-C₅ alkyl. In some variations, R₇ is n-butyl. In some variations, R₇ is n-propyl. In some variations, R₇ is ethyl. In some variations, R₇ is methyl. In some variations, In some variations, R₇ is a saturated C₅-C₁₀ cycloalkyl. In some variations, R₇ is cyclohexyl. In some variations, R₇ is a saturated straight chain C₁-C₁₀ alkoxy. In some variations, R₇ is -OC₂H₅, -OC₄H₉, or -OC₅H₁₁.

In some variations, the compound has the structure of Formula (V): 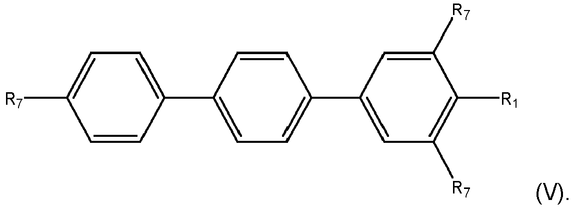 wherein R₁, R₂, and R₃ can be as described herein, in any combination, and R₈ is a saturated alkyl. In some variations, R₈ is a saturated straight chain C₁-C₁₀ alkyl. In some variations, R₈ is a saturated straight chain C₁-C₅ alkyl. In some variations, R₈ is n-butyl. In some variations, R₈ is n-propyl. In some variations, R₈ is ethyl. In some variations, R₈ is methyl. In some variations, R₈ is a saturated C₅-C₁₀ cycloalkyl. In some variations, R₈ is cyclohexyl. In some variations, R₈ is a saturated straight chain C₁-C₁₀ alkoxy. In some variations, R₈ is -OC₂H₅, -OC₄H₉, or -OC₅H₁₁.

In some variations, the compound has the structure of Formula (IV): 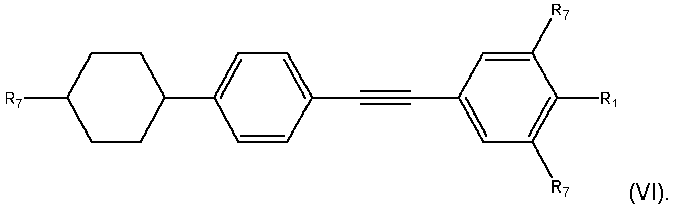 wherein R₁, R₂, and R₃ can be as described herein, in any combination, and R₉ is a saturated alkyl. In some variations, R₉ is a saturated straight chain C₁-C₁₀ alkyl. In some variations, R₉ is a saturated straight chain C₁-C₅ alkyl. In some variations, R₉ is n-butyl. In some variations, R₉ is n-propyl. In some variations, R₉ is ethyl. In some variations, R₉ is methyl. some variations, R₉ is a saturated C₅-C₁₀ cycloalkyl. In some variations, R₉ is cyclohexyl.

### Intramolecular Properties

The compounds of the disclosure have properties that improve their use in liquid crystals.

### Rigid Core and Bridging Group

The rigid core containing phenyl substituents and optional pi bond bridges can provide higher polarizability. Connecting multiple pi bonds in succession can provide for delocalized electrons over a longer intramolecular distance. Larger number of phenyl substituents and pi-containing bridges can increase the polarizability of the compound. The delocalized electrons thereby can result in a super-linear increase in polarizability of the compound.

The difference in refractive index along the long axis and transverse axis of the compound corresponds to the difference in polarizability. Increased polarizability results in increased tunability of the liquid crystal.

Figure 6 depicts the band gap of the change in polarizability for several example compounds. Absorption in the visible spectrum occurs at a band gap of 3.1 eV or less. Increasing the number of phenyl rings in the described compounds results in a smaller band gap. For example, in the compound of Formula (I), increasing variables 'm' and 'n' provide for increased polarizability, while remaining above the 3.1 eV threshold such that liquid crystal layer comprising the compound or composition remains transparent. When the pi bond bridge becomes too long, the compound can absorb radiation in the visible spectrum and in turn the liquid crystal can lose transparency in the visible spectrum. As the compounds increase in length, they can cease to be transparent. By limiting net size while increasing polarizability, the compounds and compositions are transparent through the entire visible range.

A macroscopic change in refractive index can be achieved by rotation of the molecule. As such, the relative long axis to the transverse axis corresponds to the polarizability of light incident on the liquid crystal. This property can be measured as Δn for wavelengths in the visible spectrum, determined by difference in polarizability along the long axis versus the transverse axis.

### Polar Groups

A polar groups can be at the terminal end of the compounds of the disclosure (e.g., a halogen or pseudohalogen as R₁ in the compounds of Formula (I)), creating a permanent dipole moment of the compound. In some variations, the polar group can be a halogen. In some variations, the polar group can be a pseudohalogen. The polar group can be a cyano moiety, or alternatively the polar group can be a thioisocyanate moiety.

In some variations, the polar group is an NCS moiety. The NCS moiety remains polarizable, but has a reduced permanent dipole moment, thereby having a lower likelihood of forming dipole-dipole interactions. Dimerization results in increased viscosity and consequent worse responsiveness when used as a liquid crystal-based high-speed tunable lens, for example. In some variations, the compounds of the disclosure include optional transverse moieties (e.g., R₂ and R₃ in the compounds of Formula (I)). These transverse moieties are outside central axis of the compound. The transverse moieties provide a repulsive force that increases the space between compounds, thereby further decreasing viscosity.

### Terminal group

As described herein, saturated terminal substituents opposite the polar group (e.g. substituent R₅ in Formula (II), R₆ in Formula (III), R₇ in Formula (IV), R₈ in Formula (V), or R₉ in Formula (VI)) can increase intermolecular distances between compounds, thereby further reducing intermolecular interactions in the composition. In some variations, the non-polar terminal group is a saturated alkyl group. In further variations, the saturated alkyl group can be a C₁₋₁₀ saturated alkyl group or alternatively a C₁₋₅ saturated alkyl group. In further variations, the alkyl group can be a C₁₋₁₀ saturated cycloalkyl group, for example a cyclohexyl group. The length or size of the saturated alkyl group can reduce the viscosity of the compound or composition. Reducing viscosity of the compounds when used as a liquid crystal improves the response speed of liquid crystal tunable lens in the visible spectrum. Reduced viscosity also can provide for a larger tuning range and aperture of the lens given the same figure of merit in the visible spectrum, as discussed herein.

### Compositions

In some variations, the disclosure is directed to a composition including multiple compounds described herein. By combining multiple compounds into a composition, the melting point of the liquid crystal can be reduced such that the liquid crystal composition has a lower melting point. Further, the clearing temperature can be increased such that the composition does not become isotropic.

Any number of compounds can be used in the composition. In some non-limiting variations, the composition can include any number between 1 - 20 compounds disclosed herein. In some variations, the composition can include any number between 1-15 compounds disclosed herein. In some variations, the composition can include any number between 1-10 compounds disclosed herein. In some variations, the composition can include any number between 1-5 compounds disclosed herein. The relative amounts of different compounds in the composition can be in any amount, and do not have to be in equal amounts.

The melting temperature of the liquid crystal can be further reduced by combining different compounds of the disclosure to form the disclosed compositions. The resulting eutectic composition has a lower effective melting point temperature as compared to the melting point of a single compound. The composition can include multiple compounds having different chemical compositions.

By reducing intermolecular interactions between compounds, the viscosity and melting point of the composition can be reduced, while maintaining or increasing the clearing point. Reducing viscosity while increasing the rotational elastic constant (K33) provides for reduced response time of the liquid crystal materials. Intermolecular interactions that increase viscosity include dipole-dipole interactions. Smaller compounds (e.g., two rings) can reduce the melting point and reduce viscosity. Larger compounds with delocalized electrons over a longer intramolecular distance can increase the polarization of the composition.

In some variations, the polar group can be selected to reduce dimerization of the compounds. With reference to the compound of Formula (I), R₁ can be a substituent such as thioisocyanate, which is polarizable but has a reduced permanent dipole moment, and therefore and lower likelihood of forming dipole-dipole interactions.

In the composition, different compounds with different levels of electron delocalization are provided. Different types of compositions defined by Formulae (IV), (V), and (VI) are in the composition. Different combinations of transverse substituents R₂ and R₃ reduce intermolecular interactions by providing a repulsive force to other molecules.
Tables 1A - 1C show a combination of a non-limiting example composition.

**Table 1A**

| **Number** | **Compound** | **R7** | **R2** | **R3** | **Mole Percent** |
|---|---|---|---|---|---|
| 1 | Formula (IV) | C₂H₅ | F | H | 21% |
| 2 | Formula (IV) | C₄H₉ | F | H | 23% |
| 3 | Formula (IV) | C₅H₁₁ | F | H | 7% |
| 4 | Formula (IV) | C₂H₅O | F | F | 5% |
| 5 | Formula (IV) | C₄H₉O | F | F | 6% |
| 6 | Formula (IV) | C₅H₁₁O | F | F | 5% |

**Table 1B**

| **Number** | **Compound** | **R₈** | **R₂** | **R₃** | **Mole Percent** |
|---|---|---|---|---|---|
| 7 | Formula (V) | C₃H₇ | F | F | 6% |
| 8 | Formula (V) | C₅H₁₁ | F | F | 5% |

**Table 1C**

| **Number** | **Compound** | **R₈** | **R₂** | **R₃** | **Mole Percent** |
|---|---|---|---|---|---|
| 7 | Formula (VI) | C₂H₅ | F | H | 8% |
| 8 | Formula (VI) | C₄H₉ | F | H | 14% |

In some variations, the composition includes a compound having the structure of Formula (IV), a compound having the structure of Formula (V), and a compound having the structure of Formula (VI) as described herein. In further variations, the composition includes more than one compound having the structure of Formula (IV), compound having the structure of Formula (V), and/or compound having the structure of Formula (VI).

In some variations, in the compound having the structure of Formula (IV), R₁ is NCS, R₂ is H or F, R₃ is H or F, and R₇ is selected from C₂H₅, C₄H₉, and C₂H₅O. In some variations, R₂ is F. In further variations, in the compound having the structure of Formula (IV), R₁ is NCS, R₂ is F, R₃ is H or F, and R₇ is selected from -OC₂H₅, -OC₄H₉, and -OC₅H₁₁. In further variations, the composition includes from 1- 6 of the compounds of Table 1A. In still further variations, the composition includes each compound of Table 1A.

In some variations, in the compound having the structure of Formula (V), R₁ is NCS, R₂ is H or F, R₃ is H or F, and R₈ is selected from C₃H₇ and C₅H₁₁. In some variations, R₂ and R₃ are F. In further variations, in the compound having the structure of Formula (V), R₁ is NCS, R₂ is F, R₃ is H or F, and R₈ is selected from C₃H₇ and C₅H₁₁. In further variations, the composition includes two compounds having the structure of Formula (V), wherein in the first compound R₁ is NCS, R₂ and R₃ are F, and R₈ is C₃H₇, and in the second compound R₁ is NCS, R₂ and R₃ are F, and R₈ is C₅H₁₁ (as in Table 1B).

In some variations, in the compound having the structure of Formula (VI), R₁ is NCS, R₂ is H or F, R₃ is H or F, and R₉ is selected from C₂H₅ and C₄H₉. In some variations, R₂ is F and R₃ is H or F. In some variations, R₂ is F and R₃ is H. In further variations, the composition includes two compounds having the structure of Formula (VI), wherein in the first compound R₁ is NCS, R₂ is F, R₃ is H, and R₉ is C₂H₅, and in the second compound R₁ is NCS, R₂ is F, R₃ is H, and R₉ is C₄H₉ (as in Table 1C).

### Liquid Crystal Materials

As described herein, individual compounds in the composition have reduced intermolecular interactions, a reduced permanent dipole moment as compared to substituents with a CN polar substituent, and increased intermolecular distances. The combination of these properties reduces the rotational viscosity γ1, while increasing the K33 and Δn (refractive index proportional to polarizability) in the visible spectrum to improve the response speed and tunability of liquid crystal tunable lenses. The reduced rotational viscosity also can provide for a larger tuning range and aperture of the lens.

Table 2 provides a comparison of the properties of the composition of Tables 1A - 1C to two conventional Reference Compositions 1 and 2.

**Table 2**

| **Property** | **Reference Composition 1** | **Reference Composition 2** | **Liquid Crystal Composition of Table 1A-C** |
|---|---|---|---|
| Δn | 0.257 | 0.2188 | 0.39 |
| γ1 (mPa sec, rotational velocity) | 160 | 233 | 190 |
| K33 (pN, Bend) | 14.9 | 17.1 | 29.8 |
| Figure of Merit (µm²s⁻¹) | 6.151 | 3.513 | 23.8 |

Δn corresponds to the net change in refractive index between long axis and transverse axis of the molecules. The Δn of 0.39 for wavelengths in the visible spectrum is substantial increase over Reference Compositions 1 and 2.

In some variations, the compounds and compositions have a higher K33. A higher K33 improves tunability of the lens. A higher K33 also has a larger energy barrier for thermal fluctuation, thereby reducing scattering of light incident on the liquid crystal materials that include the compounds or compositions in the visible spectrum. The K33 of 29.8 is substantially increased over Reference Compositions 1 and 2.

The compounds and compositions have a lower rotational viscosity than the composition of Tables 1A-1C. Lower rotational viscosity allows faster tuning of the liquid crystal.

The Figure of Merit (FoM) in the visible spectrum for liquid crystal ophthalmic lenses, which is equal to Δn²*K33/y1, is substantially larger than the composition described in Table 1A-1C than for conventional Reference Compositions 1 and 2 for wavelengths in the visible spectrum.

Having described several embodiments, it will be recognized by those skilled in the art that various modifications, alternative constructions, and equivalents can be used without departing from the spirit of the disclosure. Additionally, a number of well-known processes and elements have not been described in order to avoid unnecessarily obscuring the embodiments disclosed herein. Accordingly, the above description should not be taken as limiting the scope of the document.

Those skilled in the art will appreciate that the presently disclosed embodiments teach by way of example and not by limitation. Therefore, the matter contained in the above description or shown in the accompanying drawings should be interpreted as illustrative and not in a limiting sense. The following claims are intended to cover all generic and specific features described herein, as well as all statements of the scope of the method and system, which, as a matter of language, might be said to fall there between.

### Numbered Statements of Invention

1. A compound having the structure of Formula (I): 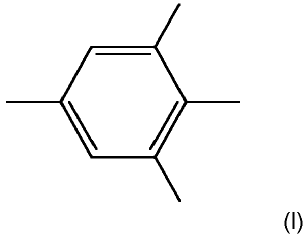 wherein
   R₁ is selected from hydrogen, a saturated C₁₋₁₀ alkyl, halogen, and pseudohalogen;
   R₂ and R₃ are each independently selected from hydrogen, a halogen, and a pseudohalogen;
   R₄ is selected from Formula (II) and Formula (III): 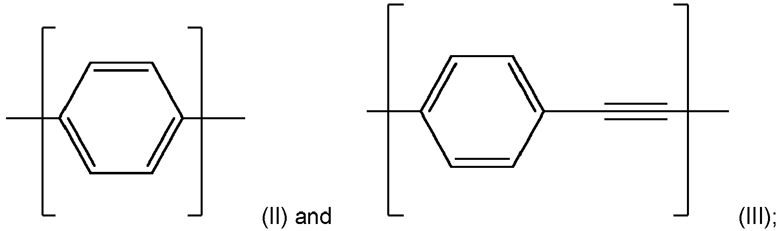
   m is an integer from 1 to 5;
   R₅ is saturated C₁-C₁₀ alkyl or saturated C₁-C₁₀ alkoxy;
   n is an integer from 1 to 5; and
   R₆ is saturated C₁-C₁₀ alkyl or saturated C₁-C₁₀ alkoxy.
2. The compound of statement 1, selected from a structure of Formulae (IV), (V), and (VI): 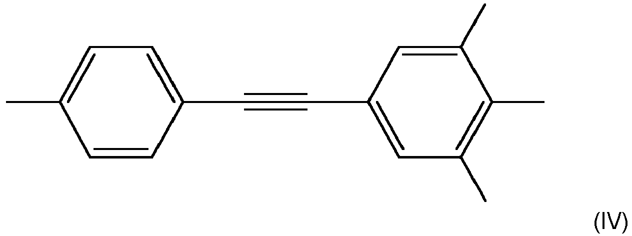 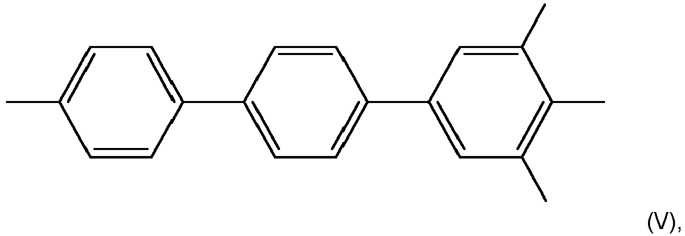 and 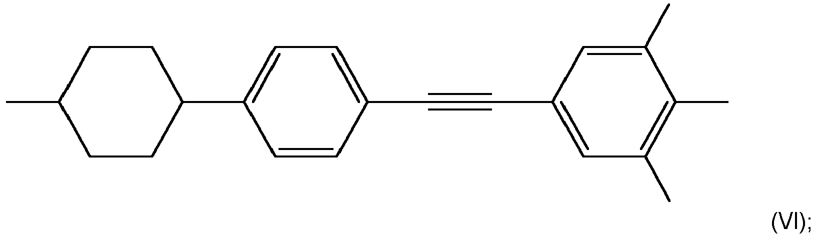 wherein
   R₁ is selected from hydrogen, a saturated C₁₋₁₀ alkyl, a halogen, and a pseudohalogen;
   R₂ and R₃ are each independently selected from hydrogen, a halogen, and a pseudohalogen;
   R₇ is saturated C₁-C₁₀ alkyl or saturated C₁-C₁₀ alkoxy;
   R₈ is saturated C₁-C₁₀ alkyl or saturated C₁-C₁₀ alkoxy; and
   R₉ is saturated C₁-C₁₀ alkyl.
3. The compound of statement 2, wherein the compound has the structure of Formula (IV).
4. The compound of statement 3, wherein R₁ is a pseudohalogen.
5. The compound of statement 4, wherein R₁ is NCS.
6. The compound of any one of statements 3-5, wherein R₂ and R₃ are each independently selected from H and F.
7. The compound of statement 6, wherein R₂ and R₃ are both F.
8. The compound of statement 6, wherein R₂ is F and R₃ is H.
9. The compound of any one of statements 3-8, wherein R₇ is selected from C₂H₅, C₄H₉, and C₂H₅O.
10. The compound of statement 9, wherein R₇ is C₂H₅.
11. The compound of statement 9, wherein R₇ is C₄H₉.
12. The compound of statement 9, wherein R₇ is C₂H₅O.
13. The compound of statement 2, wherein the compound has the structure of Formula (V).
14. The compound of statement 13, wherein R₁ is a pseudohalogen.
15. The compound of statement 14, wherein R₁ is NCS.
16. The compound of any one of statements 13-15, wherein R₂ and R₃ are each independently selected from H and F.
17. The compound of statement 16, wherein R₂ and R₃ are both F.
18. The compound of any one of statements 13-17, wherein R₈ is selected from C₃H₇ and C₅H₁₁.
19. The compound of statement 18, wherein R₈ is C₃H₇.
20. The compound of statement 18, wherein R₈ is C₅H₁₁.
21. The compound of statement 2, wherein the compound has the structure of Formula (VI).
22. The compound of statement 21, wherein R₁ is a pseudohalogen.
23. The compound of statement 22, wherein R₁ is NCS.
24. The compound of any one of statements 21-23, wherein R₂ and R₃ are each independently selected from H and F.
25. The compound of statement 24, wherein R₂ is H and R₃ is F.
26. The compound of any one of statements 21-25, wherein R₉ is selected from C₂H₅ and C₄H₉.
27. The compound of statement 26, wherein R₉ is C₂H₅.
28. The compound of statement 26, wherein R₉ is C₄H₉.
29. A composition comprising a first compound according to statement 1, and a second compound of statement 1, wherein the first and second compounds are different.
30. The composition of statement 29, comprising the first compound according to one of statements 2-12, the second compound according to one of statements 13-20, and a third compound according to one of statements 21-28.
31. The composition of statement 29, comprising compound 1-6 of Table 1A, compound 7-8 of Table 1B, and compound 9-10 of Table 1C.
32. A liquid crystal cell comprising:
   a first transparent substrate and a second transparent substrate on opposing sides of a liquid crystal layer,
   the liquid crystal layer comprising a compound according to any one of statements 1-28 or a composition according to any one of statements 29-31.
33. The liquid crystal lens comprising a first liquid crystal cell, a second liquid crystal cell, and a third liquid crystal cell, each of the first, second and third liquid crystal cells according to statement 32.

## Claims

1. A compound having the structure of Formula (I): 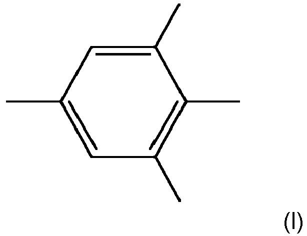 wherein
R₁ is selected from hydrogen, a saturated C₁₋₁₀ alkyl, halogen, and pseudohalogen;
R₂ and R₃ are each independently selected from hydrogen, a halogen, and a pseudohalogen;
R₄ is selected from Formula (II) and Formula (III): 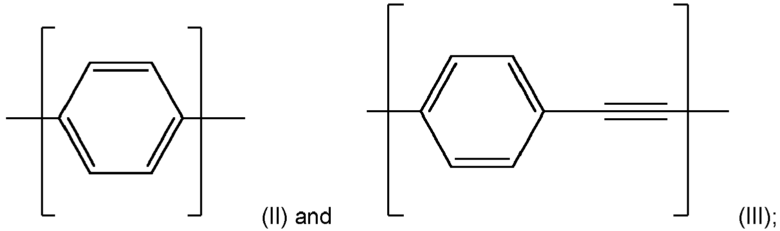
m is an integer from 1 to 5;
R₅ is saturated C₁-C₁₀ alkyl or saturated C₁-C₁₀ alkoxy;
n is an integer from 1 to 5; and
R₆ is saturated C₁-C₁₀ alkyl or saturated C₁-C₁₀ alkoxy.

2. The compound of claim 1, selected from a structure of Formulae (IV), (V), and (VI): 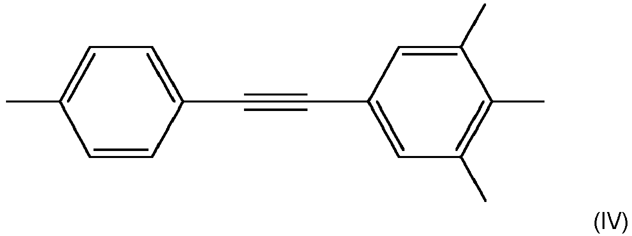 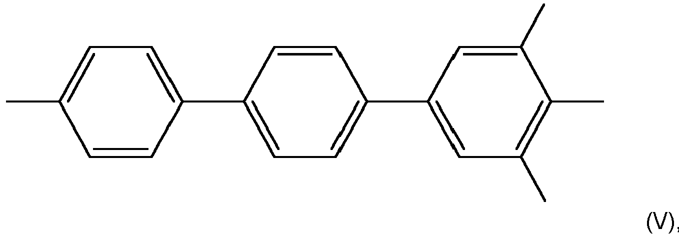 , and 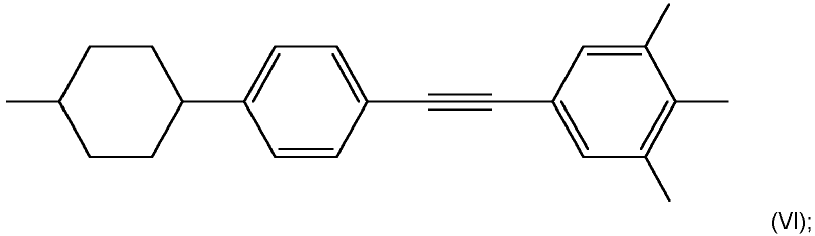 wherein
R₁ is selected from hydrogen, a saturated C₁₋₁₀ alkyl, a halogen, and a pseudohalogen;
R₂ and R₃ are each independently selected from hydrogen, a halogen, and a pseudohalogen;
R₇ is saturated C₁-C₁₀ alkyl or saturated C₁-C₁₀ alkoxy;
R₈ is saturated C₁-C₁₀ alkyl or saturated C₁-C₁₀ alkoxy; and
R₉ is saturated C₁-C₁₀ alkyl.

3. The compound of claim 2, wherein the compound has the structure of Formula (IV), and preferably
wherein R₁ is a pseudohalogen, and more preferably wherein R₁ is NCS.

4. The compound of claim 3 wherein R₂ and R₃ are each independently selected from H and F, and preferably wherein R₂ and R₃ are both F, or wherein R₂ is F and R₃ is H.

5. The compound of any one of claims 3-4, wherein R₇ is selected from C₂H₅, C₄H₉, and C₂H₅O; and preferably wherein R₇ is C₂H₅, or wherein R₇ is C₄H₉, or wherein R₇ is C₂H₅O.

6. The compound of claim 2, wherein the compound has the structure of Formula (V), and preferably wherein R₁ is a pseudohalogen, and more preferably wherein R₁ is NCS.

7. The compound of any one of claims 5-6, wherein R₂ and R₃ are each independently selected from H and F; and preferably wherein R₂ and R₃ are both F.

8. The compound of any one of claims 6-7, wherein R₈ is selected from C₃H₇ and C₅H₁₁; and preferably wherein R₈ is C₃H₇, or wherein R₈ is C₅H₁₁.

9. The compound of claim 2, wherein the compound has the structure of Formula (VI), and preferably wherein R₁ is a pseudohalogen and more preferably wherein R₁ is NCS.

10. The compound of claim 9, wherein R₂ and R₃ are each independently selected from H and F; preferably wherein R₂ is H and R₃ is F.

11. The compound of any one of claims 9-10, wherein R₉ is selected from C₂H₅ and C₄H₉; preferably wherein R₉ is C₂H₅ or wherein R₉ is C₄H₉.

12. A composition comprising a first compound according to claim 1, and a second compound of claim 1, wherein the first and second compounds are different.

13. The composition of claim 12, comprising the first compound according to any one of claims 2-5, the second compound according to any one of claims 6-8, and a third compound according to any one of claims 9-11.

14. The composition of claim 12, comprising compound 1-6 of Table 1A, compound 7-8 of Table 1B, and compound 9-10 of Table 1C.

15. A liquid crystal cell comprising:
a first transparent substrate and a second transparent substrate on opposing sides of a liquid crystal layer,
the liquid crystal layer comprising a compound according to any one of claims 1-11 or a composition according to any one of claims 12-14.

16. The liquid crystal lens comprising a first liquid crystal cell, a second liquid crystal cell, and a third liquid crystal cell, each of the first, second and third liquid crystal cells according to claim 15.
